# EUROPEAN PATENT APPLICATION

(11) **EP 3 195 881 A1**
(43) Date of publication of application: **26.07.2017**
(21) Application number: 16152005.1
(22) Date of filing: 20.01.2016
(51) Int. Cl.: A61K 45/06, A61K 31/4365, A61P 17/00

(54) **SUBSTANCE THAT INHIBITS P2Y12 RECEPTOR, FOR USE IN THE PREVENTIVE TREATMENT OF SYSTEMIC SCLEROSIS IN PATIENTS WITH RAYNAUD'S PHENOMENON AND A DYSIMMUNITY**

(71) Applicant: Université de Bordeaux, 33000 Bordeaux (FR); CENTRE NATIONAL DE LA RECHERCHE SCIENTIFIQUE -CNRS-, 75794 Paris Cedex 16 (FR); Centre Hospitalier Universitaire de Bordeaux, 33404 Talence (FR)
(72) Inventor: TRUCHETET, Marie-Élise, 33000 BORDEAUX (FR); CONTIN-BORDES, Cécile, 33700 MÉRIGNAC (FR); BLANCO, Patrick, 33490 VERDELAIS (FR); SCHAEVERBEKE, Thierry, 33200 BORDEAUX (FR); BARNETCHE, Thomas, 33330 SAINT-ÉMILION (FR)
(74) Representative: Novagraaf Technologies

(57) **Abstract**

The present invention relates to a substance that inhibits P2Y12 receptor for use in the preventive treatment of systemic sclerosis in patients with Raynaud's phenomenon and a dysimmunity.

## Description

### Technical field

The present invention refers to a substance for use in the preventive treatment of systemic sclerosis.

Therefore, the present invention has utility in the medical and pharmaceutical fields.

In the description below, the references into brackets ([ ]) refer to the listing of references situated at the end of the text.

### Background of the Invention

Systemic sclerosis (SSc) is a complex systemic autoimmune disease classically classified in two subsets: limited cutaneous (ISSc) and diffuse cutaneous (dSSc), characterized by microvascular dysfunction, immune activation and interstitial and perivascular fibrosis affecting the skin and internal organs (Gabrielli et al. N Engl J Med 2009; 360:1989-2003 **([1])).** The slow progress of the disease is accompanied by increasing functional disability. Complications involving life-threatening may occur, particularly pulmonary fibrosis. The prevalence is estimated at 10 cases / 100 000. The disease usually occurs between 40 and 50 years with no causes clearly identified.

Currently, no specific cure is available when the disease has occurred (Tyndall et al.: "Causes and risk factors for death in systemic sclerosis a study from the EULAR Scleroderma Trials and Research (EUSTAR) database", Ann Rheum Dis 2010; 69: 1809-1815 **([2])).**

In systemic sclerosis (SSc), the link between vascular damage and fibrosis remains largely unknown. More particularly, the way in which the processes of microvascular dysfunction, immune activation and interstitial and perivascular fibrosis are inter-related is currently unknown, and a better understanding of the interplay between the three pathologic hallmarks is likely to contribute to a better knowledge of the disease pathogenesis and therefore to improve treatment options.

The fact that activation of endothelial cells (EC) appears very early during the course of the disease suggests that chronic fibroblast activation might result, at least in part, to endothelial dysfunctions **(([1]);** Pattanaik et al.: "Pathogenesis of Systemic Sclerosis", Front Immunol 2015; 6:272 **([3])).**

For over 25 years, many observations have highlighted platelet activation in patients with declared SSc. It has been demonstrated that microvascular endothelium activation precede intimal hyperplasia characteristic of vascular remodelling in SSc (Prescott et al.: "Sequential dermal microvascular and perivascular changes in the development of scleroderma", J Pathol 1992; 166:255-263 **([4])).** The progressive loss of the vascular architecture is supposed to be involved in accumulation of extracellular matrix and chronic activation of platelets observed in SSc patients (Kahaleh MB, Osborn I, Leroy EC. Elevated levels of circulating platelet aggregates and beta-thromboglobulin in scleroderma. Ann Intern Med 1982;96:610-613 **([5]) ;** Postlethwaite AE, Chiang TM. Platelet contributions to the pathogenesis of systemic sclerosis. Curr Opin Rheumatol 2007;19:574-579 **([6]) ;** Solanilia A, Villeneuve J, Auguste P, et al. The transport of high amounts of vascular endothelial growth factor by blood platelets underlines their potential contribution in systemic sclerosis angiogenesis. Rheumatology (Oxford) 2009;48:1036-1044 **([7]) ;** Chiang TM, Takayama H, Postlethwaite AE. Increase in platelet non-integrin type I collagen receptor in patients with systemic sclerosis. Thromb Res 2006;117:299-306 **([8])).** Conversely, platelet derived-factors modulate endothelial cells (EC) activation and homeostasis in SSc (Maugeri N, Franchini S, Campana L, et al. Circulating platelets as a source of the damage-associated molecular pattern HMGB1 in patients with systemic sclerosis. Autoimmunity 2012;45:584-587 **([9]);** van Bon L, Affandi AJ, Broen J, et al. Proteome-wide analysis and CXCL4 as a biomarker in systemic sclerosis. N Engl J Med 2014; 370:433-443 **([10])).** Recently, the role of platelet-derived serotonin in fibroblast activation and extracellular matrix component deposition has been described in SSc (Dees C, Akhmetshina A, Zerr P, et al. Platelet-derived serotonin links vascular disease and tissue fibrosis. J Exp Med 2011; 208:961-972 **([11])).** However, whether platelets/EC interaction could induce the secretion of factors directly involved in fibroblast activation and subsequently in fibrosis remains uncertain in SSc.

It follows from the foregoing that several platelet soluble mediators are increased in the plasma of SSc patients. Among these mediators, there are beta-thromboglobulin, platelet factor 4 (PF-4), platelet-derived growth factor (PDGF), soluble CD40L or Vascular Endothelial Growth Factor (VEGF) (Postlethwaite et al. **([6]) ;** Kahaleh et al.: **([5]) ;** Allanore et al.: "Increased plasma soluble CD40 ligand concentrations in systemic sclerosis and association with pulmonary arterial hypertension and digital ulcers", Ann Rheum Dis 2005; 64: 481-483 **([12]) ;** Solanilla al. **([7])).** Platelets of SSc patients also over-express activation membrane markers such as P-selectine (Solanilla et al. **([7])).**

Platelet activation in SSc patients is also accompanied by the translocation from the cytoplasm to the membrane of a alarmine: HMGB1 (High Mobility to Group Box 1) capable of inducing necrosis of endothelial cells (Maugeri et al. **([9])).** Circulating levels of HMGB1 are increased in SSc patients and correlate positively to Rodnan score and negatively to lung function (Yoshizaki et al.: "Clinical significance of serum HMGB-1 and sRAGE levels in systemic sclerosis: association with disease severity", J Clin Immunol 2009;29: 1 80-189 **([13])).**

A recent study has highlighted the major role of CxCL4 (PF-4), especially produced by platelets in the pathophysiology of SSc. In 2014, the group of T. Radstake showed that the increase of circulating levels of CxCL4 was a biomarker of SSc (van Bon et al. **([10])).** Indeed, CxCL4 is increased during the early phase of the disease and is responsible for the increased production of ET-1. It disrupts the vascular formation, maturation, and stabilization, notably through the inhibition of VEGF.

Analysis of beta-thromboglobulin and CxCL4 rates in bronchoalveolar fluid of 37 SSc patients showed increased levels in nearly 30% of patients, and only in SSc patients with recent interstitial lung disease suggesting active role of platelets in pulmonary fibrosis (Kowal-Bielecka et al.: "Beta thromboglobulin and platelet factor 4 in bronchoalveolar lavage fluid of patients with systemic sclerosis", Ann Rheum Dis 2005;64:484-486 **([14])).**

Finally, the increase in circulating levels of serotonin by excessive release of the mediator by activated platelets had been described in patients SSc (Klimiuk et al.: "Platelet serotonin in systemic sclerosis", Ann Rheum Dis 1989;48:586-589 **([15]));** the demonstration of its pro-fibrotic effect in scleroderma has been demonstrated only much more recently (Dees et al.: **([11])).** Serotonin release by activated platelets causes the excessive production of extracellular matrix by fibroblasts in SSc patients, in a TGF-β signal-dependent manner. In the same publication, the *in vivo* blocking of platelet activation and of the release of serotonin by the clopidogrel reduces fibrosis in two models mice model of scleroderma providing proof of concept for the therapeutic benefit of the control of platelet activation but only in patients with SSc reported (Dees et al.: **([11])).**

Despite all these data on physiopathology of SSc disease, there is no effective treatment of this disease, and the molecules currently used for the treatment of the disease reported, as cyclophosphamide, which is an immunosuppressor, show a very moderate success and a risk of substantial side effects for the patient.

The bibliographic data in patients at risk of developing SSc are far fewer than the ones for patients having an SSc.

A key step in the development of SSc is the preclinical phase or stage of pre-disease. It has been shown that especially the immune and vascular anomalies are observable several years before the development of clinical disease. Among these anomalies, two are particularly relevant and easy to detect upstream of SSc:
- Raynaud's phenomenon (RP): this is an overreaction of blood microcirculation to some stimuli such as cold. Alone, this phenomenon can be a physical trait without any clinical consequences, but some RP are the first manifestation of SSc. There would be in the RP excessive platelet activation that could have effects well beyond the classical hemostasis, affecting vasculopathy, dysimmunity and tissue remodeling that are observed in SSc (Pauling et al.: "The contribution of platelets to the pathogenesis of Raynaud's phenomenon and systemic sclerosis", Platelets 2013; 24: 503-515 **([16])).** Subjects simply having Raynaud's phenomenon have increased platelet activation markers (Silveri et al.: "Relative rates of endothelial cell damage and platelet activation in primary Raynaud's phenomenon (RP) and RP secondary to systemic sclerosis", Scand J Rheumatol 2001; 30: 290-296 **([17])).** In patients identified as having pre-scleroderma, some markers of endothelial activation (endothelin-1 (ET-1) and Inter Cellular Adhesion Molecule (ICAM -1) soluble) and platelet activation (beta-thromboglobuline and Platelet-Denved Growth Factor (PDGF)) were found increased **(([17]) ;** Vettori et al.: "Early systemic sclerosis: serum profiling of factors involved in endothelial, T-cell, and fibroblast interplay is marked by elevated interleukin-33 levels", J Clin Immunol 2014; 34: 663-668 **([18]));**
- Dysimmunity: during the preclinical phase, autoimmunity intensifies and autoantibodies can be detected and measured while no clinical signs can allow warning the subject.

The preclinical phase, also called "pre-SSc state", is so defined in three groups' according to the presence of a RP, a dysimmunity and/or abnormal capillaroscopy without any of other criteria for the classification of SSc based on the 2013 ACR/EULAR (American College of Rheumatology /European League Against Rheumatism) criteria. Group I is defined as the combination of a RP, a specific dysimmunity, with the presence notably of anti-ScI70, anti-centromere, anti-RNA polymerase III (anti-RNA pol III), anti-Th/To and/or anti-PM-ScI (polymyositis-scleroderma) antibodies, and abnormal capillaroscopy. In these subjects, the risk of moving towards a genuine SSc reaches 80% within 3 years. Group II, includes subjects with RP and specific dysimmunity without abnormal capillaroscopy. In this group, the risk of moving forward SSc ranges from 35% to 66% within 3 years. Finally, the group III which associates RP, non-specific dysimmunity (i.e. anti-nuclear antibodies other than the aforementioned auto-antibodies) and abnormal capillaroscopy, has 25% risk to developp SSc within 3 years (Koenig et al.: "Autoantibodies and Microvascular Damage Are Independent Predictive Factors for the Progression of Raynaud's Phenomenon to Systemic Sclerosis », ARTHRITIS & RHEUMATISM, Vol. 58, No. 12, December 2008, pp 3902-3912 **([42]);** Valentini et al.: "Early Systemic Sclerosis: Analysis of the Disease Course in Patients With Marker Autoantibody and/or Capillaroscopic Positivity", Arthritis Care & Research, Vol. 66, No. 10, October 2014, pp 1520-1527 **([43])).**

This preclinical phase could be a window of opportunity for treatment to prevent progression to scleroderma, at three conditions:
- that it is relatively simple, inexpensive and it has a favorable risk-benefit ratio for the patient,
- that a clear physiopathologic mechanism is identified on which it would be possible to act early,
- that one can identify the target population, as it does not present significant clinical signs in the majority of cases.

The individual advantage for the patient would be significant since SSc is usually a life-threatening condition in medium term, and with no specific cure when the disease has occurred.

Thus, a need exists of alternative treatments, more efficient and less toxic treatments of SSc, in particular preventive treatment of SSc. The present invention fulfills these and other needs.

### Description of the invention

After extensive researches on series of systemic sclerosis, the present Applicants are the first ones to have experimented and observed that platelet activation may contribute to fibrosis in patients SSc through endothelial activation. These observations make a link between two key elements of the pathophysiology of SSc: vasculopathy and fibrosis. The Applicants are the first ones to show that *in vitro* platelet activation induces the production of a pro-fibrotic mediator, the Thymic Stromal Lymphopoietin (TSLP), by dermal microvascular endothelial human cells in a mechanism dependent on the release of IL-1β and platelet serotonin, and that the blockade of this platelet activation inhibits the expression of TSLP by endothelial cells.

The Applicants also surprisingly demonstrated that TSLP allows inducing the secretion of collagen A1 and A2 and the decrease of production of metalloprotease (MMP-1 and MMP-2) by matrix dermal fibroblasts directing them to a pro-fibrotic profile.

These *in vitro* observations are confirmed *in vivo* since the Applicants surprisingly highlighted an overexpression of TSLP in the dermis of SSc patients, largely due to endothelial cells. TSLP was also expressed by keratinocytes but was fairly homogenous across the SSc population. The Applicants surprisingly demonstrated in particular that the expression of dermis TSLP expression in SSc patients correlates with the Rodnan score. The dermal expression of TSLP was also inversely correlated with disease duration (since the appearance of non-Raynaud phenomenon (RP) symptoms). Finally, dermal TSLP levels were higher in patients with pulmonary arterial hypertension (PAH) diagnosed with ultrasonography (PAPS>35 mmHg).

Thus the Applicants' results show for the first time TSLP production by endothelial cells under the influence of platelet derivatives and its potential role in the observed fibrosis in patients SSc. These results thus underlie that the blockage of platelet activation could prevent the fibrotic process in SSc patients.

The Applicants have thus surprisingly shown the relationship between platelet activation, endothelial cell production of TSLP and fibrosis.

Thus, the invention is to provide a preventive treatment in patients at risk of developing systemic scleroderma. On the pathophysiological data the Applicants have obtained, the proposed treatment is the use of an inhibitor of P2Y12 receptor, in order to prevent the occurrence of SSc in these patients.

This strategy would greatly reduce the socio-economic burden of SSc disease that is very important right now. In addition, this new indication could encourage the early detection of these subjects at risk as part of primary care by offering a preventive therapeutic action. General practitioners would be more attentive to identify such patients if they had a preventive action to offer them.

Thus, the invention generally relates to an inhibitor of P2Y12 receptor, or a mixture of two or more inhibitors of P2Y12 receptor, for use in the preventive treatment of systemic sclerosis in patients with Raynaud's phenomenon and a dysimmunity.

P2Y12 receptor, also named P2Y₁₂, P2Y purinoceptor 12, ADP-glucose receptor, ADPG-R, P2T(AC), P2Y(AC), P2Y(cyc), P2Y12 platelet ADP receptor, P2Y(ADP), SP1999, P2RY12, is encoded by the P2RY12 (also named HORK3) gene. It belongs to the Gi class of a group of G protein-coupled (GPCR) purinergic receptors and is a chemoreceptor for adenosine diphosphate (ADP). The P2Y family has several receptor subtypes with different pharmacological selectivity, which overlaps in some cases, for various adenosine and uridine nucleotides. P2Y12 is found mainly but not exclusively on the surface of blood platelets, and is an important regulator in blood clotting via platelet aggregation.

"Inhibitor of P2Y12 receptor" refers herein to any antagonist of P2Y12 receptor that is likely to limit or to avoid, reversibly or irreversibly, selectively or not selectively, the biological normal effect of P2Y12 receptor. More particularly, it may refer to any antagonist of P2Y12 receptor, that is likely to bind, reversibly or irreversibly, P2Y12 receptor, thereby limiting or avoiding, reversibly or irreversibly, the biological normal effect of P2Y12 receptor. By "limit" or "limiting" it is understood a decrease of at least 50%, for example 60%, 70%, 80%, 90% or 99%, of the biological effect of P2Y12 receptor compared to the normal biological effect of P2Y12 receptor, i.e. the biological effect of P2Y12 receptor that is not inhibited by the inhibitor.

The inhibitor of P2Y12 receptor may be selected among the group of substances comprising thienopyridines, ticagrelor, elinogrel and cangrelor, , their active metabolites, pharmaceutical salts thereof and a mixture thereof.

Advantageously, the substance may be selected among a thienopyridine, an active metabolite of a thienopyridine and a pharmaceutical salt thereof. For example, the thienopyridine may be selected among the group comprising clopidogrel, prasugrel and ticlopidine.

Thienopyridines are a class of selective, irreversible P2Y12 inhibitors, generally used for their anti-platelet activity. Drugs in this class are clopidogrel, available on the market as Plavix™, prasugrel, available on the market as Effient™ and ticlopidine, available on the market as Ticlid™. All the thienopyridines are prodrugs that are rapidly metabolized to a pharmacologically active metabolite and inactive metabolites. Thienopyridine has the chemical formula represented below (Formula I):

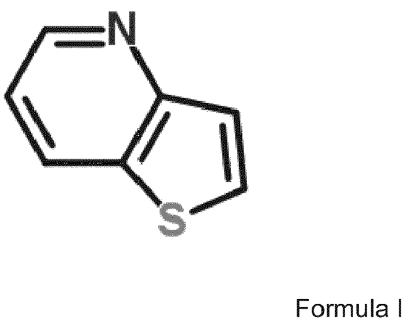

Pharmaceutical salt of thienopyridines may be chosen among hydrogen sulphate, besylate, bisulfate, hydrochloride, resinate, napadisilate, hydrochloride, hydrobromide, phosphate and nitrate.

Clopidogrel (International Nonproprietary Name) is a thienopyridine-class antiplatelet agent that is generally used to inhibit blood clots in coronary artery disease, peripheral vascular disease, cerebrovascular disease, and to prevent myocardial infarction, especially heart attack. Clopidogrel works by irreversibly inhibiting P2Y12 receptor. It is a prodrug, which requires cytochrome P450 2C19 (CYP2C19) for its activation to an active metabolite in the liver. The active metabolite of clopidogrel contains a thiol group which binds to a free cysteine on the P2RY12 receptor and irreversibly blocks ADP binding and receptor activation. Clopidogrel has the chemical formula represented below (Formula II):

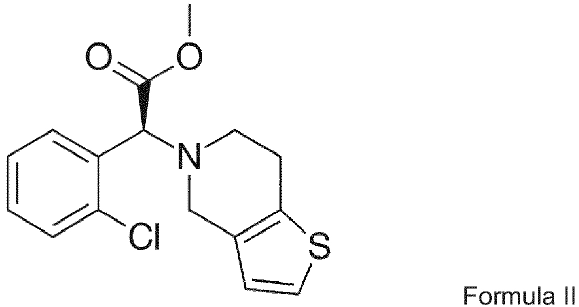

Clopidogrel is a ticlopidine analogue and is thus structurally related to ticlopidine, the first member of this class of antiplatelet agents. Clopidogrel was developed to get backup compounds with a better benefit/risk ratio in humans compared to ticlopidine. Prasugrel is a thienopyridine of third generation developed in the same goal (Jean-Pierre Maffrand: "The story of clopidogrel and its predecessor, ticlopidine: Could these major antiplatelet and antithrombotic drugs be discovered and developed today?", C. R. Chimie 15 (2012) 737-743 **([44])).**

Clopidogrel may be administered as a base. Alternatively, it may be administered as a pharmaceutical salt of clopidogrel that may be chosen among the group comprising hydrogen sulphate, besylate, bisulfate, hydrochloride, hydrobromide, resinate and napadisilate.

Prasugrel (International Nonproprietary Name) is a thienopyridine-class antiplatelet agent that is generally used to reduce the aggregation of platelets by irreversibly binding to P2Y12 receptors. Prasugrel is a prodrug that undergoes rapid hydrolysis in the intestine to a thiolactone, which is then converted to the active metabolite, primarily by CYP3A4 and CYP2B6, and to a lesser extent by CYP2C9 and CYP2C19. The active metabolite is metabolized to 2 inactive compounds by S-methylation or conjugation with cysteine. Prasugrel is available as a racemate comprising equal amounts of (R)- and (S)-prasugrel, and has the chemical formula represented below (Formula III):

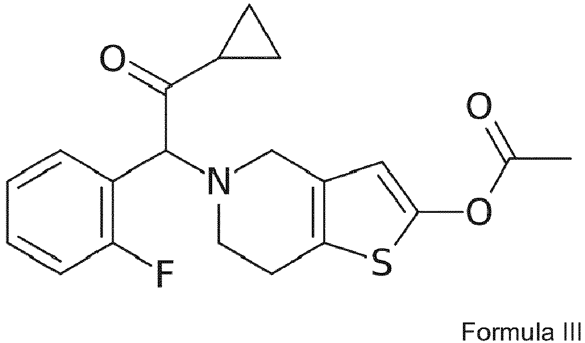

Prasugrel may alternatively by used as an active purified enantiomer.

Pharmaceutical salt of prasugrel may be chosen among the group comprising hydrochloride, hydrobromide, phosphate, nitrate.

Ticlopidine (International Nonproprietary Name) is a thienopyridine-class antiplatelet agent which is an adenosine diphosphate (ADP) receptor inhibitor. It is a prodrug that is metabolized to active thiol metabolites in the liver, which blocks the ADP receptor that is involved in GPIIb/IIIa receptor activation leading to platelet aggregation. Ticlopidine has the chemical formula represented below (Formula IV):

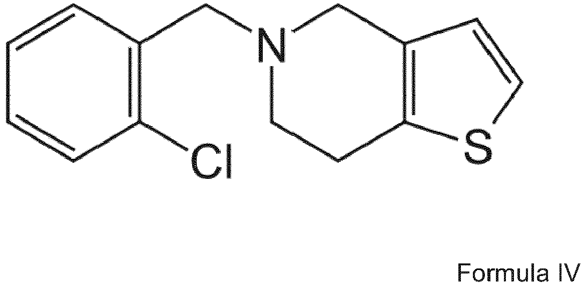

Pharmaceutical salt of ticlopidine may be chosen among the group comprising hydrochloride.

Ticagrelor is a cyclo-pentyltriazolo-pyrimidine that reversibly inhibits the P2Y12 receptor. It is a nucleoside analogue, in which the cyclopentane ring is similar to the sugar ribose, and the nitrogen rich aromatic ring system resembles the nucleobase purine, giving the molecule an overall similarity to adenosine. Unlike the thienopyridines prasugrel, clopidogrel and ticlopidine, ticagrelor has a binding site different from ADP, making it an allosteric antagonist, and the blockage is reversible. Ticagrelor has one active metabolite, which is AR-C124910XX, formed by O-deethylation, but ticagrelor and its active metabolite are approximately equipotent. Ticagrelor has the chemical formula represented below (Formula V):

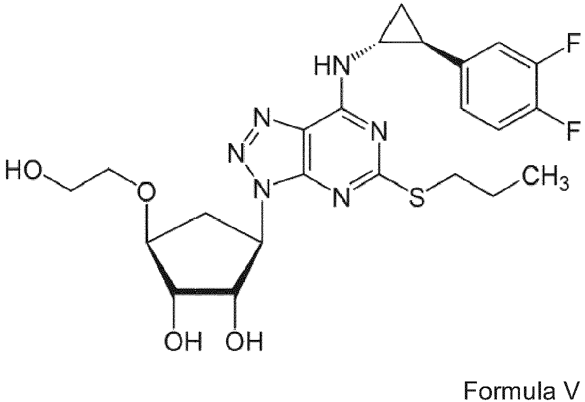

Ticagrelor may exist in a number of different substantially crystalline forms (Polymorph I, Polymorph II, Polymorph III and Polymorph IV).

Cangrelor is a P2Y12 reversible inhibitor antiplatelet drug, and is an active drug not requiring metabolic conversion. Cangrelor has the chemical formula represented below (Formula VI):

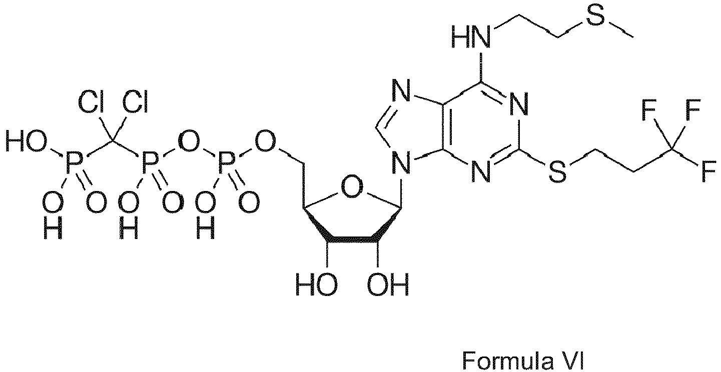

Elinogrel is an antiplatelet drug acting as a P2Y12 inhibitor. Similarly to ticagrelor, elinogrel is a reversible inhibitor. It is pharmacologically active itself. Elinogrel has the chemical formula represented below (Formula VII):

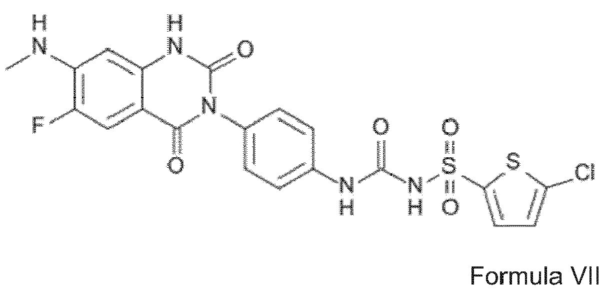

Elinogrel may be used in the form of its potassium salt, for example orally.

More particularly, the invention relates to a substance selected among thienopyridines, their active metabolites, pharmaceutical salts thereof and a mixture thereof, for use in the preventive treatment of systemic sclerosis in patients with Raynaud's phenomenon and a dysimmunity.

Even more particularly, the substance may be chosen among clopidogrel and prasugrel.

Preferentially, the substance used is chosen among the group comprising clopidogrel, its pharmaceutical salts, its active metabolites and a mixture thereof.

Clopidogrel, just as aspirin, is an antiplatelet agent. However, in contrast to aspirin, clopidogrel induces in addition a decrease of the exocytosis of the platelet soluble mediators and of membrane expression of co-stimulatory molecules of the immune response as CD40L. The Applicants have shown that unlike aspirin, clopidogrel can block the pro-fibrotic TSLP molecule expression *in vitro* from healthy endothelial cells. Without being linked by any biology mechanism theory, the Applicants have hypothesized, in light of these results and their experimental results regarding the link between platelet activation and fibrosis via the production, by endothelial cells, of TSLP, that these immunological aspects of clopidogrel, i.e. this immunomodulatory effect of clopidogrel, in addition of the hemostasis effect of clopidogrel, may undergo, at least in part, its effect in the preventive treatment of systemic sclerosis in patients with Raynaud's phenomenon and a dysimmunity, via the blockade of platelet activation and the inhibition of the expression of TSLP by endothelial cells.

"Preventive treatment" refers herein to the prophylactic measures taken for disease prevention, as opposed to disease treatment. More particularly, a preventive treatment is a treatment administered to a patient at risk to develop, in the future, systemic sclerosis, for the purpose of delaying, limiting or preventing the onset of systemic sclerosis in the patients at risk.

"Patients at risk" refers herein to any patients currently having or having had, in the past, a Raynaud's phenomenon and a dysimmunity, with eventually a capillaroscopy with scleroderma pattern.

According to the present invention, "preventing" means that the preventive treatment of the invention results in a lack, within 2 years after the start of the treatment, of at least one symptom chosen in the group comprising cutaneous sclerosis, digital ulcer, dyspnea at stage 3 or 4, abnormal capillaroscopy, heartburn resisting to one month of a conventional treatment, joint pain and/or joint swelling, telangiectasias, sub-cutaneous calcinosis, pigmentation disorders and myalgia.

According to the present invention, "delaying" means that the preventive treatment results in that the period between the occurrence of Raynaud's phenomenon and/or a dysimmunity and/or capillaroscopy with scleroderma pattern and the occurrence of SSc is in average longer in the patients treated by the preventive treatment of the invention than the period in the patients not treated by the preventive treatment of the invention. The period in a treated patient may be longer of more than one year, for example two years, or three years, or more, comparing to the mean period in non-treated patients.

According to the present invention, "limiting" means that the seriousness of the symptoms, and/or the scope of the symptoms, is (are) in average decreased by the preventive treatment of the invention.

"Raynaud's phenomenon" refers herein to a reduced blood flow in response to cold or emotional stress, causing at least one of the following symptoms: discoloration of the fingers, toes, and occasionally other areas, nails becoming brittle with longitudinal ridges, and atrophy of the skin, subcutaneous tissues, and muscle. Without being linked by a mechanistic theory, these symptoms can be caused by a hyperactivation of the sympathetic nervous system causing extreme vasoconstriction of the peripheral blood vessels, leading to tissue hypoxia. The diagnosis may be realized by any set of diagnostic criteria currently available to the man skilled in the art, for example by the description of the symptoms by the patient, and then confirmed by a careful medical history made by a physician.

"Dysimmunity" refers herein to an abnormal autoimmunity, especially the presence of a positive level of autoantibodies that can be detected and/or measured while no other clinical signs can be detected. Dysimmunity may be a specific dysimmunity or a non-specific dysimmunity.

"Specific dysimmunity" refers herein to antinuclear antibodies detectable both in SSc patients and in some subjects with high risk for SSc (the aforementioned I and II groups). They may include anti-centromere (A and/or B), anti-DNA topoisomerase I (also called anti-ScI70), anti-RNA polymerase III, anti-Th/To and/or anti-Pm-ScI.

"Non-specific dysimmunity" refers herein to antinuclear antibodies detectable both in subjects with weak risk for SSc (the aforementioned group III). They may include autoantibodies that are not specific of SSc, and so that are different than the specific autoantibodies. The autoantibodies may be in this case antinuclear antibodies (ANA). The presence of autoantibodies, and particularly ANA, may be measured by any method currently available, indirect immunofluorescence on Hep-2 cells being the gold standard for ANA detection. International guidelines for ANA detection state that significant positive ANA level are equal or above 1/160 for adult, 1/320 for people above 65 years old and 1/40 for children (Agmon-Levin N. et L.: "International recommendations for the assessment of autoantibodies to cellular antigens referred to as anti-nuclear antibodies.", Ann Rheum Dis. 2014 Jan;73(1):17-23 **([45])).**

"Capillaroscopy with scleroderma pattern" refers herein to the presence of larger capillaries (megacapillaries) and/or avascular areas and/or microhaemorrhages on nailfold, compared to a healthy subject, revealed by morphological assessments of the microcirculation. Advantageously, capillaroscopy may be a videocapillaroscopy.

Advantageously, the substance as defined above may inhibit platelet activation. "Inhibit" refers herein to a decrease of platelet activation to a normal level, or nearly normal level of platelet activation, i.e. a level of platelet activation occurring in a healthy subject. The decrease of platelet activation may be a decrease of at least one of the platelet activation marker chosen among CD40L, IL-1β, serotonin, beta-thromboglobulin, CxCL4 (PF-4). The decrease may be a decrease of at least 20%, for example 30%, or 40%, or 50%, or 60%, or 70%, or 80%, or 90% or more.

Advantageously, the substance as defined above may inhibit the expression and secretion of at least one pro-inflammatory factor resulting from platelet activation. "Inhibit" refers herein to a decrease of expression and secretion of pro-inflammatory factors resulting from platelet activation to a normal level, or nearly normal level of platelet activation, i.e. a level of expression and secretion of pro-inflammatory factors resulting from platelet activation occurring in a healthy subject. The pro-inflammatory factor may be chosen among CD40L, IL-1β, serotonin, beta-thromboglobulin, CxCL4 (PF-4). The decrease may be a decrease of at least 20%, for example 30%, or 40%, or 50%, or 60%, or 70%, or 80%, or 90% or more.

Advantageously, the substance as defined above may inhibit the expression and/or production of Thymic Stromal Lymphopoietin (TSLP) by endothelial cells. More particularly, the expression and/or secretion of TSLP by endothelial cells may occur in a mechanism dependent on the release of IL-1β and platelet serotonin by activated platelet. TSLP is an interleukin-7 (IL-7) cytokine family member that has been implicated in various inflammatory disorders (Hillen MR, Radstake TR, Hack CE, et al. Thymic stromal lymphopoietin as a novel mediator amplifying immunopathology in rheumatic disease (Rheumatology (Oxford) 2015 **([19]);** Lo Kuan E, Ziegler SF. Thymic stromal lymphopoietin and cancer. J Immunol 2014; 193:4283-4288 **([20])).** TSLP promotes the differentiation of naïve T-cell into a Th2 phenotype, and the secretion of various pro-fibrotic factors including IL13 (Ito T, Wang YH, Duramad O, et al. TSLP-activated dendritic cells induce an inflammatory T helper type 2 cell response through OX40 ligand. J Exp Med 2005; 202:1213-1223 **([21]);** Pedroza-Gonzalez A, Xu K, Wu TC, et al. Thymic stromal lymphopoietin fosters human breast tumor growth by promoting type 2 inflammation. J Exp Med 2011; 208:479-490 **([22])).** Indeed, the Applicants demonstrate that *in vitro* platelet activation induces the production of TSLP by dermal microvascular endothelial human cells in a mechanism dependent on the release of IL-1β and platelet serotonin.

The substance may be administered to a patient at risk as defined above at a pharmaceutically acceptable and efficient dose for the preventive treatment of the invention.

The substance may be administered as long as necessary, for example for period equal at or longer than 2 years, or 5 years, or 10 years, or 20 years, or 30 years, or for life. For example, clopidogrel may be administered for life.

For example, the substance may be administered to a patient at risk by administration of a dose comprised of from 5 mg to 250 mg per day, for example of from 10 mg to 200 mg, or from 50 mg to 150 mg.

For example, clopidogrel may be administered at a dose of about 75mg per day, on the form of a single dose per day. In this embodiment the dose may be comprise of from 50mg per day to 100mg per day, or of from 70mg to 90mg per day.

In another example, prasugrel may be administered at a dose of about 5mg, once or twice per day, or at a dose of about 10mg once per day. In this embodiment the dose may be comprised of from 2mg per day to 20mg per day, or of from 7mg to 15mg per day.

In another example, ticlopidine may be administered at a dose of about 250 mg once per day. In this embodiment the dose may be comprised of from 150mg per day to 300mg per day, or of from 180mg to 280mg per day.

In another example, ticagrelor may be administered at a dose of about 90mg once per day. In this embodiment the dose may be comprised of from 50mg per day to 120mg per day, or of from 70mg to 100mg per day.

In one embodiment, the administration may be an oral administration. In this embodiment, it may be a buccal or a sublingual administration. It may for example be in the form selected from the group comprising a liquid formulation, an oral effervescent dosage form, an oral powder, a pill, a multiparticule system, an orodispersible dosage form, a solution, a syrup, a suspension, an emulsion and oral drops. When the medicament is in the form of an oral effervescent dosage form, it may be in a form selected from the group comprising tablets, granules, powders. When the medicament is the form of an oral powder or a multiparticulate system, it may be in a form selected from the group comprising beads, granules, mini tablets and micro granules. When the medicament is the form of an orodispersible dosage form, it may be in a form selected from the group comprising orodispersible tablets, lyophilized wafers, thin films, a chewable tablet, a tablet and a capsule, a medical chewing gum. According to the present invention, when the medicament is for buccal and sublingual routes, it may be selected from the group comprising buccal or sublingual tablets, muco adhesive preparation, oro-mucosal drops and sprays.

In another embodiment, the administration is a parenteral administration. The parenteral administration may be selected from the group comprising intravenous administration, intramuscular administration and subcutaneous administration. In those cases, the substance may be in the form of an injectable solution.

In another embodiment, the administration may be a transdermal or a transmucosal administration. When the medicament is for topical-transdermal administration, it may be selected from the group comprising ointments, cream, gel, lotion, patch and foam. It may also be a medicament for nasal administration, for example selected from the group comprising nasal drops, nasal spray, nasal powder.

The skilled person in the art understands clearly that the term "form" as used herein refers to the pharmaceutical formulation of the medicament for its practical use. For example, the medicament may be in a form selected from the group comprising a tablet (for example as Plavix® 75mg, or Effient® 5mg or 10mg, or Ticlid® 250 mg, or Brilique® 90mg), an injectable form (for example as Avlocardyl®5mg/ml), syrup (for example as Efferalgan^{®}3%), oral suspension (for example as Efferalgan^{®}3%), a pellet (for example as Dafalgan^{®}1g), powder (for example as Doliprane^{®}100mg), granules (for example as Zoltum^{®}10mg), spray, transdermal patch (for example as Cordipatch^{®}5mg/24h) or local form (cream, lotion, collyrium) (for example as Dermoval creme^{®}, as Betneval^{®}lotion and as Chibroxine^{®} collyre respectively).

In these examples, the substance, for example clopidogrel, may be added or may replace, when relevant, the active ingredient(s) of said medicaments.

The pharmaceutically acceptable carrier may be any known suitable pharmaceutically carrier used for the administration of a substance to a human or to an animal, depending on the subject. For example, this carrier may be one or more carrier(s) present in Plavix^{®}, or in Effient^{®} or in Ticlid^{®}, or in Brilique^{®}.

This invention is further illustrated by the following examples with regard to the annexed drawings that should not be construed as limiting.

### Brief description of the figures

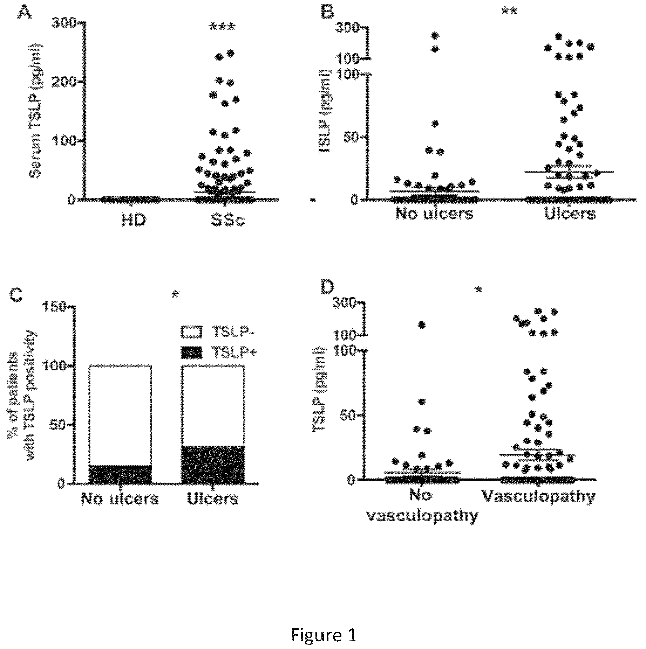

- **Figure 1****:** represents the demonstration of the overexpression of TSLP in serum of scleroderma patients especially in those with digital ulcers. **(A)** TSLP measurements in serum of 20 healthy donors (HD), 203 scleroderma (SSc) patients. A multiple comparison non-parametric test of Kruskal Wallis was used to compare TSLP serum levels. **(B)** TSLP serum levels in SSc patients according to the presence of digital ulcers. An unpaired t-test was used. **(C)** Percentage of patients with TSLP positivity in serum according to the presence or absence of digital ulcers. A Fisher's exact test was used. **(D)** TSLP serum levels in SSc patients according to vascular profile comprising either digital ulcer, PAH scleroderma and/or renal crisis. An unpaired t-test was used. In scatter dot plot, each dot represents a separate individual. P<0.05 was considered significant.

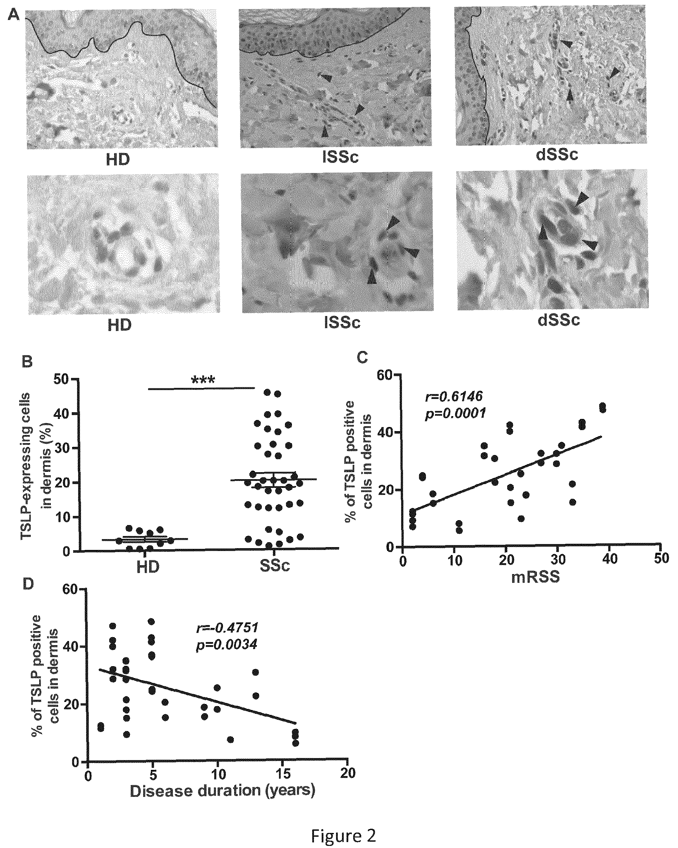

- **Figure 2****:** represents the demonstration that TSLP is overexpressed in SSc patient skin compared to healthy donors and correlates to fibrosis. **(A)** Immunohistochemistry (IHC) was used to identify ex vivo expression of TSLP in human skin. IHC representative staining of TSLP in one out of 5 healthy donors (HD), 9 limited cutaneous SSc (ISSc) and 10 diffuse cutaneous SSc (dSSc) are shown (Original magnification x40). Arrowheads show positive TSLP staining. On the lower panel IHC of representative staining of TSLP with an x100 magnification showing the perivascular distribution of the positive cells. Arrowheads show positive TSLP staining. **(B)** Quantification of TSLP positive dermal cells, expressed as number of TSLP positive cells in the dermis among total cells in the analyzed section for 2 separate slides from 5 different HD and 18 SSc skin. p<0.0001, using unpaired t-test. **(C)** Correlation between the percentage of dermal TSLP expressing cells and the skin fibrosis score of Rodnan (mRSS) (*p=0.0001 and r=0.6146 using Spearman test). **(D)** Correlation between the percentage of dermal TSLP expressing cells and the disease duration in years (*p=0.0034 and r=0.4751 using Spearman test). Cellular count was analysed using ImageJ software.

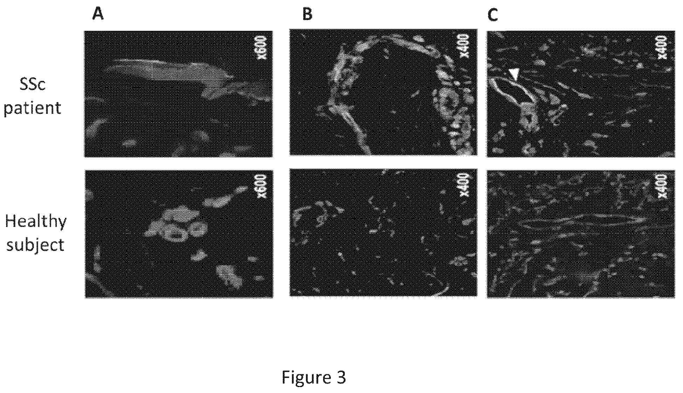

- **Figure 3****:** represents the demonstration that endothelial cells are able to produce TSLP *ex vivo* in SSc and *in vitro.* Indirect immunofluorescence analysis of TSLP in skin of healthy donors (lower panels) and SSc patient (upper panels) showing **(A, B)** co-staining of TSLP (green) and αSMA (red) for fibroblasts **(A)** and pericytes **(B), (C)** co-staining of TSLP (green) and CD31 (red) for endothelial cells. Skin sections were analyzed with an epifluorescence microscope (Olympus AX70). Nuclei were DAPI stained (blue). Original magnification x400 or x600 as indicated. Yellow color shows co-stained cells.

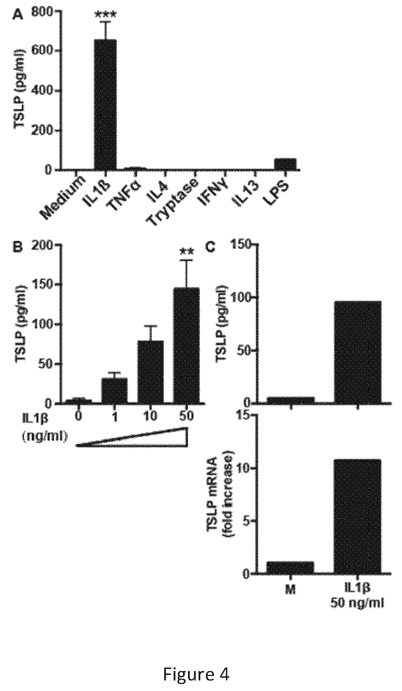

**Figure 4****:** IL1β-stimulated human dermal microvascular endothelial cells (HDMEC) produce TSLP. Normal HDMEC were purified from normal skin (plastic surgery) and stimulated 24 hrs with **(A)** cytokines potentially involved in SSc and the TLR4 agonist LPS or **(B)** increasing amount of IL1β (from 10 to 50 ng/ml) in complete MV-2 medium (M). Supernatants were harvested and TSLP content was measured by dedicated ELISA from eBioscience. Mean and SD from 3 **(A)** and 6 **(B)** independent experiments. **(C)** Quantification of TSLP mRNA from IL1β (50 ng/mL) activated HDMEC using RTqPCR, one representative experiment out of 3. *P<0.05 and ***P=0.0004 using Kruskal Wallis test.

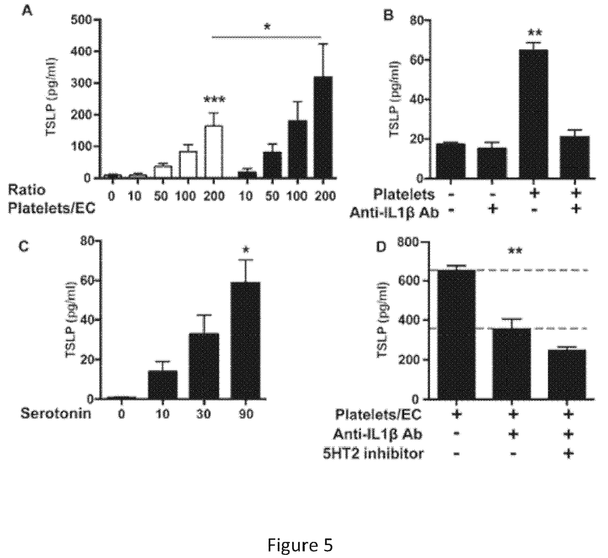

- **Figure 5****:** represents the demonstration that platelets induce TSLP production by human dermal microvascular EC (HDMEC) in an IL1β-dependent manner. **(A)** 70000 HDMEC were cultured for 24hrs in 24-well culture plate in MV-2 complete medium along with increasing amount of purified non-activated platelets (white bars) or adenosine diphosphate-activated platelets (black bars) (from 10 to 200 platelets for 1 HDMEC). A multiple comparison non-parametric test of Kruskal Wallis was used to compare TSLP levels (***p<0.0001). **(B)** HDMEC were stimulated with ADP-activated platelets with or without anti-IL1β blocking antibody. Condition "platelet -" is a comparison without platelets. A multiple comparison non-parametric test of Kruskal Wallis was used to compare TSLP levels (**p=0.007). **(C)** HDMEC were stimulated with increasing doses of serotonin (from 0 to 90 ng/ml). A Wilcoxon test was used. (*p=0.013). **(D)** HDMEC were stimulated with ADP-activated platelets (ratio HDMEC:platelets=1:200) with or without blocking anti-lL1β with or without 5HT-receptors (5HTR2a and 5HTR2b) blocking antibodies. A multiple comparison non-parametric test of Kruskal Wallis was used to compare TSLP levels (**p=0.007). Supernatants were harvested and TSLP content was measured using dedicated ELISA. Results are mean and SEM from 3 independent experiments.

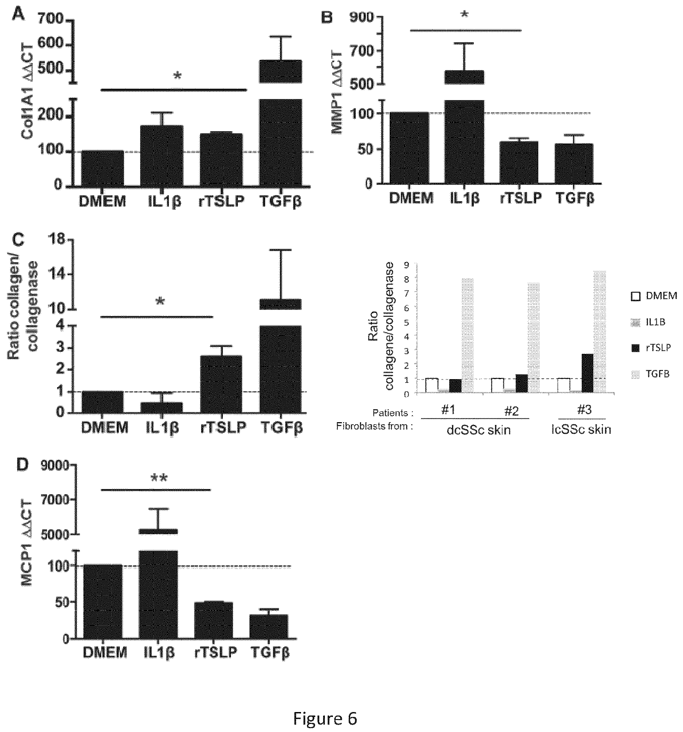

**Figure 6****:** represents the demonstration that TSLP induces a pro-fibrotic profile in dermal fibroblasts from healthy donors and SSc patients. Human dermal fibroblasts were isolated from healthy skin and incubated 48h with DMEM, IL1β, recombinant TSLP or TGFβ1. **(A)** Quantification of Col1A1 mRNA from activated fibroblasts using RT-qPCR from 3 different healthy donors was expressed in ΔΔCT with DMEM as the baseline condition. **(B)** Quantification of MMP-1 mRNA from activated fibroblasts using RTqPCR, results of 3 different healthy donors. **(C)** Ratio of COL1A1 on MMP-1 (collagenase) mRNA from activated normal fibroblasts using RT-qPCR, results of 3 different healthy donors (left panel) and 3 SSc patients (right panel). Among SSc patients, two patients were dcSSc and fibroblasts came from TSLP+ fibrotic skin (patients #1 and 2), the other was a IcSS and fibroblasts came from TSLP- low fibrotic skin (patient #3).. **(D)** Quantification of MCP-1 from activated normal fibroblasts using RT-qPCR, results of 3 different donors. Kruskal Wallis test was used to compare the mean of mRNA expression levels. *P<0.05.

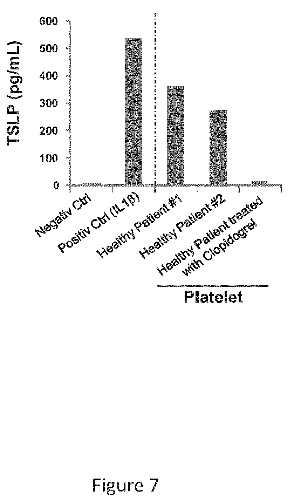

- **Figure 7****:** represents the *in vitro* TSLP production (pg/ml) by healthy microvascular endothelial cells cultured in the presence of (from left to right): a negative control (medium alone without platelet), a positive control (healthy endothelial cells treated with 50 ng/mL IL-1β), platelets from a first healthy donor, platelets from a second healthy donor and platelets from a non sclerodermic patient treated with Clopidogrel (75 mg/day). In all conditions, ADP (adenosine di-phosphate) was added to induce platelet activation.

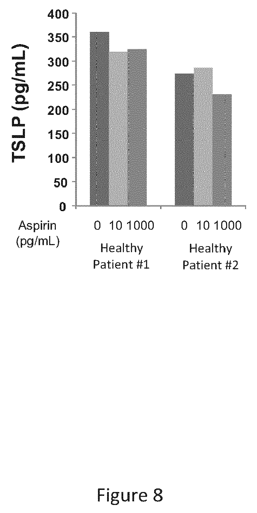

- **Figure 8****:** represents the *in vitro* TSLP production (pg/ml) by healthy endothelial cells in the presence of platelets from a first healthy non sclerodermic patient a second non sclerodermic patient previously treated with acetylsalicylic acid (aspirin, pg/ml) at a dose of 0, 10 and 1000 pg/ml.

### Examples

### Example 1: patients and methods

**Study population.** 203 SSc individuals presenting at the University hospital of Bordeaux, France were prospectively included in the study between March 2012 and January 2015. All patients satisfied the classification criteria proposed by the American College of Rheumatology (ACR) and the European League Against Rheumatism (EULAR) 2013 (van den Hoogen F, Khanna D, Fransen J, et al. 2013 classification criteria for systemic sclerosis: an American College of Rheumatology/European League against Rheumatism collaborative initiative. Arthritis Rheum 2013; 65:2737-2747 **([23])).** Patients were included in the context of the VISS (Vasculopathy and Inflammation in Systemic Sclerosis) biomedical research project founded in 2012 and approved by the ethical committee (CPP, 2012-A00081-42, Aquitaine). All participants gave written informed consent before their inclusion.

**Cell cultures.** Fibroblasts were obtained from lesional skin biopsy samples from 3 healthy donors as described elsewhere (Truchetet ME, Brembilla NC, Montanari E, et al. Interleukin-17A+ cell counts are increased in systemic sclerosis skin and their number is inversely correlated with the extent of skin involvement. Arthritis Rheum 2013;65:1347-1356 **([24])).**

**Immunohistochemistry and cell quantification.** Immunohistochemistry was performed as described (Truchetet et al. **([24])).** Epitope damasking was performed heating in a bath at 98° for 40 min in 10mM Tris buffer/1mM EDTA, pH 9. Immunohistochemical images were acquired by scanning the whole tissue sections using the Nanozoomer apparatus from the Bordeaux Imaging Centre (BIC). At least two sections for each skin biopsy were analyzed per individual. Each section was manually subdivided in epidermis and dermis using the ImageJ software (for detailed information see http://rsbweb.nih.gov/ij/). Annexes were excluded from the analysis. TSLP+ cells in the dermis were semi-automatically quantified using ImageJ software. Positive cells (brown) were filtered for size and normalized to the total number of cells (blue) in each field. Representative images were manually reviewed by 2 operators (MET and BD).

**Immunofluorescence.** After paraffin removal, epitope retrieval and blocking in PBS-4% BSA, tissue sections were incubated with anti-TSLP (sheep, AF1398, R&D), anti-CD31 (rabbit, SAB4502167, Sigma-Aldrich) and anti-alpha-SMA (clone 1A4, DAKO), antibodies. The binding was revealed with Alexa-488-conjugated donkey anti-goat and alexa-568 conjugated donkey anti-mouse or anti-rabbit from Invitrogen (Oslo, Norway). Nuclei were stained with DAPI. Laser-scanning confocal images were acquired using LSM510meta microscope (Zeiss).

**Quantitative real-time PCR.** Total RNA was isolated from trypsinized fibroblasts with the NucleoSpin RNA II extraction system (Macherey-Nagel, Duren, Germany) and cDNA synthesized from 0.25 µg of total RNA using random hexamers and Superscript III reverse transcriptase (Invitrogen, Carlsbad, CA) according to manufacturer's instructions. Gene expression was quantified using SYBR green performed on a SDS 7900 HT instrument (Applied Biosystems). Specific primer pairs for each gene described in supplementary material (Datta A, Alexander R, Sulikowski MG, et al. Evidence for a functional thymic stromal lymphopoietin signaling axis in fibrotic lung disease. J Immunol 2013;191:4867-4879 **([25]) ;** Brembilla NC, Montanari E, Truchetet ME, et al. Th17 cells favor inflammatory responses while inhibiting type I collagen deposition by dermal fibroblasts: differential effects in healthy and systemic sclerosis fibroblasts. Arthritis Res Ther 2013;15:R151 **([26]) ;** Yoda A, Yoda Y, Chiaretti S, et al. Functional screening identifies CRLF2 in precursor B-cell acute lymphoblastic leukemia. Proc Natl Acad Sci U S A 2010;107:252-257 **([27]) ;** Le QT, Gomez G, Zhao W, et al. Processing of human protryptase in mast cells involves cathepsins L, B, and C. J Immunol 2011;187:1912-1918 **([28])).** Each reaction was performed in triplicates. The more stable housekeeping genes (GAPDH (Glyceraldehyde-3-phosphate dehydrogenase) and EEF1A1 (eukaryotic translation elongation factor 1 alpha 1)) were selected for normalization. Oligonucleotides were obtained from Sigma-Aldrich. Differences were calculated with the threshold cycle (Ct) and the comparative Ct method for relative quantification.

**Cytokine determinations.** TSLP levels in cell culture supernatants or serum was quantified using an ELISA kit from eBiosciences following the manufacturer's instruction.

**Endothelial cells activation.** Human dermal microvascular EC were cultured in complete MV2 growth medium in a 96 well-plates with flat bottom (20000 cells per well in 200 µl) and allowed to stand overnight before adding different reagents (IL-1β, IL-4, IL-13, TNF-α, IFN-γ, LPS, 5HT or Tryptase) or platelets ADP-activated or not for 24h at 37°C. Blocking anti-IL1b (canakinumab), or anti-serotonin receptors (5HT2a and 5HT2b) were added at a 10 µg/mL final concentration when indicated. Platelets enriched plasma was prepared from whole blood using a venepuncture on EDTA tube after a 20 min centrifugation at 1000 rpm without acceleration and break. Then the supernatant was harvested and 1µM of PGE1 was added. After a centrifugation (10 min at 2000 rpm without break), supernatant was discarded and tyrode buffer was added to obtain an 8.10⁸ platelets/ml solution.

**Statistical analysis.** Statistical analyses were performed using GraphPad Prism (La Jolla, CA). For populations who satisfied the Kolmogorov-Smirnov normality test, a two-tailed Student's *t*-test for unpaired or paired samples and one-way repeated-measures ANOVA test followed by Bonferroni correction were used to compare the different populations according to the experimental design. When normality test was not satisfied Mann-Whitney, Wilcoxon and Kruskal Wallis test were used. Correlations were analysed using Spearman test. P value <0.05 was considered statistically significant.

### Example 2: results

### Circulating TSLP is increased in sera from SSc patients and is associated with digital ulcers

From April 2013 to January 2015, 203 consecutive SSc patients were included in the study. Limited form of the disease represented around 2/3 of the cohort (n=133, 65.5%). Demographic observations and main clinical features are described in Table 1 below.

**Table 1:**

| Disease characteristics of 203 patients with Systemic Sclerosis | | | | |
|---|---|---|---|---|
| | **Limited** | | **All subsets** | |
| | **cutaneous Systemic Sclerosis** | **Diffuse cutaneous Systemic Sclerosis** | | **p*** |
| | n = 133 | n=70 | n = 203 | |
| ***Clinical features*** | | | | |
| Female sex - no (%) | 111 (83.4) | 40 (57.1 ) | 151 (74.3) | <0.0001 |
| Age at onset (Raynaud) - mean ± SD | | | | |
| years | 42±12.9 (n=117) | 47.4±12.4 (n=66) | 43.9±12.8 (n=183) | 0.006 |
| Age at onset (other symptoms) - mean ± | | | | |
| SD years | 50.2±11.5 (n=130) | 50.8±12 (n=69) | 50.4±11.7 (n=199) | **0.73** |
| Disease duration (since Raynaud) - mean | | | | |
| ± SD years | 16.8±10.9 (n=117) | 11.8±7.8 (n=66) | 15±10.1 (n=183) | 0.001 |
| Disease duration (since other symptoms) | | | | |
| - mean ± SD years | 9.5±5.9 (n=130) | 8.5±5.5 (n=69) | 9.2±5.8 (n=199) | **0.24** |
| Positive test for anti nuclear antibodies - no (%) | 119(89.4) | 68(97.1) | 187 (92.1) | **0.052** |
| Anti-centromere - no (%) | 83 (62.4) | 4 (5.7) | 87 (42.8) | <0.0001 |
| Anti-Scl70 - no (%) | 2 (1.5) | 48 (68.5) | 50 (24.6) | <0.0001 |
| Raynaud- no (%) | 128 (96.2) | 69 (97.1) | 197 (97) | **0.66** |
| Digital ulcers - no (%) | 52 (39) | 48 (68.5) | 96 (47.2) | <0.0001 |
| Rodnan score - mean ± SD years | 4.7±2.6 (n=132) | 17.78 ± 9.54 | 9.2±7.3 (n=202) | <0.0001 |
| RVSP>35mmHg - no (%) | 34 (25.9) (n=131 ) | 25 (35.7) | 59 (29.3) (n=201) | **0.87** |
| Pulmonary arterial hypertension - no (%) | 8 (6.1) | 8(11.4) | 16 (7.9) (n=201) | **0.28** |
| Interstitial lung disease - no (%) | 23 (18.2) (n=126) | 50 (71.4) | 73 (37.2) (n=196) | <0.0001 |
| Lung fibrosis - no (%) | 10 (7.9) (n=126) | 29(41.4) | 39 (19.8) (n=196) | <0.0001 |
| Renal crisis - no (%) | 4 (3) | 6 (8.5) | 10 (4.9) | **0.09** |
| | | | | |

| ***Treatments - no (%)*** | | | | |
|---|---|---|---|---|
| Steroids | 50 (38.1) (n=131 ) | 51 (82.6) (n=69) | 101 (50.5) (n=200) | <0.0001 |
| Dose, median [IQR] Eq prednisone (mg) | 10 [6-30] | 15 [10-30] | 12.5 [7.5-30] | |
| Immunosuppressive therapy | 18 (13.5) | 46 (65.7) | 64 (31.5) | <0.0001 |
| Methotrexate | 29 (21.8) | 28 (40) | 57 (28) | 0.006 |
| Hydroxycholoroquine | 28 (21) | 15(21.4) | 43 (21.1) | **0.97** |
| Proton pomp inhibitors | 101 (75.9) | 63 (90) | 164 (80.7) | 0.01 |
| Calcium channel blocker | 87 (65.4) | 54 (77.1) | 141 (69.4) | **0.07** |
| Angiotensin-converting enzyme inhibitors | 30 (22.5) | 27 (38.5) | 57 (28) | 0.01 |
| Iloprost | 26 (19.5) | 27 (38.5) | 53 (26,1) | 0.002 |
| Endothelin receptor antagonist | 32 (24) | 33 (47.1) | 65 (32) | 0.0005 |
| Phosphodiesterase inhibitor | 4 (3) | 7 (1) | 11 (5.4) | **0.0509** |

| | | | | |
|---|---|---|---|---|
| * between the limited cutaneous form and the diffuse cutaneous form. | | | | |

Serum level of TSLP was assessed by ELISA. We observed that TSLP level was significantly increased in serum from SSc patients compared to HD (mean ± SEM of 12.95±2.6 pg/mL versus 0 pg/ml respectively, cf. Figure 1A, *P<0.0001, Kruskal Wallis test followed by Dunn post hoc test). We observed a trend toward higher levels of TSLP in dSSc compared to ISSc although it did not reach the significance (mean ± SEM of 19.02±5.8 pg/mL versus 12.4±3.2 pg/mL respectively). Not only patients with dSSc tend to present higher levels of TSLP than ISSc, but they also tend to be more frequently positive for TSLP (29% vs 21%, cf. Figure 1C). To determine whether TSLP could be a biomarker in SSc, we looked for a correlation between circulating TSLP and several clinical parameters. No correlation was found between serum level of TSLP and fibrotic parameters such as modified Rodnan skin score (mRSS), diffusing capacity of the lung for carbon monoxide (DLCO), or Total Lung Capacity (TLP). In the same way there was no association between disease duration or PAH diagnosed with ultrasonography. In contrast we observed a significant association between TSLP and vascular damages, as TSLP levels were significantly higher in patients with digital ulcers compared to patients without any history of digital ulcers (mean ± SEM respectively of 22.03±3.1 pg/mL vs 6.85±4.6 pg/mL, cf. Figure 1B, p=0.0089, unpaired t test). In addition, TSLP serum levels were increased in patients with at least one symptom of vasculopathy attested by the presence of digitals ulcers, and/or scleroderma renal crisis (SRC) and/or Pulmonary Arterial Hypertension (PAH) on Right Heart Catheterization (RHC) (mean ± SEM respectively of 19.48±4.2 pg/mL vs 5.53±2.6 pg/mL, cf. Figure 1D, p=0.022). Altogether these results suggest that serum TSLP is increased in SSc patients and is associated to vascular damage.

### TSLP is overexpressed in SSc patient skin compared to healthy donors and correlates to fibrosis.

In order to analyse the cellular source of TSLP we conducted IHC analysis of TSLP expression in skin from SSc patients and controls. Direct staining with secondary antibodies, did not result in any significant fluorescence (cf. Figure 2A, left panel) confirming the specificity of the staining. To confirm the specificity of the staining pre-incubation of anti-TSLP Abs with a 10x excess of TSLP recombinant proteins was performed and resulted in a virtually complete abrogation of the staining except for a slight border in the basal membrane (cf. Figure 2A, right panel). While TSLP expression was almost undetectable in the skin of healthy individuals, we observed a significant TSLP expression in both, the dSSc and ISSc, as previously described (Usategui A, Criado G, Izquierdo E, et al. A profibrotic role for thymic stromal lymphopoietin in systemic sclerosis. Ann Rheum Dis 2013 **([29])).** Interestingly, TSLP expression was detected in the epidermis and in the dermis particularly in the perivascular area of dSSc, consistent with previous report (Christmann RB, Mathes A, Affandi AJ, et al. TSLP upregulation in human SSc skin and induction of overlapping profibrotic genes and intracelullar signaling with IL-13 and TGFss. Arthritis Rheum 2013 **([30])).** These observations hold true also in ISSc patients. The staining of TSLP on the epidermis was fairly homogenous across the SSc populations; we therefore focused our attention on the cell expressing TSLP in the dermis. The numbers of total cells and TSLP positive cells were determined in the dermis section surface of biopsies. Whereas the number of total cells per mm² was not statistically different between groups (1190±138.3 cells in SSc vs 1046±98.2 cells in HD slides, p=0.4909 unpaired t-test, see Figure 2B), the proportion of dermal TSLP-positive cells was significantly increased in SSc compared to HD skin (20.35±2.28 cells vs 3.669±0.88 cells respectively, p<0.0001 unpaired t-test, see Figure 2C). A trend toward a higher percentage of TSLP positive cells in dcSSc compared to IcSSc was observed (29.92±3.2% vs 20.28±5.1%, data not shown). Dermal TSLP expression correlated to skin fibrosis as assessed by the mRSS (r=0.6146, p=0.0001 using the Spearman test). Dermal expression of TSLP was also inversely correlated with the disease duration (since non Raynaud phenomenon (RP) symptoms) (r=-0.4751, p=0.0034 using the Spearman test, see Figure 2D). Finally, dermal TSLP was higher in patients with ultrasonography PAH (PAPS>35 mmHg) (33.12±2.8% of positive cells vs 23.58±2.4% of positive cells, p=0.0238 unpaired t test).

We conclude that TSLP expression in SSc skin is increased in perivascular areas, associated with early form of the disease and correlated to the extent of skin fibrosis.

### Human dermal microvascular EC overexpress TSLP in SSc skin ex vivo and produce TSLP in vitro in an IL1β-dependent-mechanism.

To further analyse which cell type stained positive for TSLP we first used an immunofluorescence method. We observed that both the alpha-smooth muscle actin (SMA) positive myofibroblasts (see Figure 3A) and the CD31 positive EC stained positively for TSLP (see Figure 3C arrowhead). Of note, the perivascular alpha-SMA positive pericytes were negative for TSLP (see Figure 3B).

To confirm that skin EC could be a potent TSLP producer, we cultured *in vitro* dermal microvascular EC in the presence or absence of pro-fibrotic cytokines (IL-4 and IL-13), pro-inflammatory cytokines (IL1β, TNFα and IFNγ) and TLR agonists. We found that only IL1β was able to increase the expression of TSLP at the protein (651.7±95.4 pg/ml vs 7.4±3.5 pg/ml with dermal microvascular EC alone, p=0.0002 paired t testsee Figures 4B and 4C) and the transcriptomic levels. All the other stimuli tested had no effect except for the LPS that induced a slight but reproducible TSLP production (see Figure 4A).

Taken together these results demonstrate that platelets are potent inducers of TSLP production by dermal microvascular EC in an IL1β dependant manner.

### Activated platelets promote the secretion of TSLP by EC via an IL-1β and serotonin-dependent mechanism.

Among the cells activated in SSc patients and characterized by their ability to secrete IL-1β, platelets appeared of significant interest (Dees et al. **([11]) ;** Loppnow H, Bil R, Hirt S, et al. Platelet-derived interleukin-1 induces cytokine production, but not proliferation of human vascular smooth muscle cells. Blood 1998; 91:134-141 **([31])).** Platelets from healthy volunteers induced a TSLP production by dermal microvascular EC (164.7±41.4 pg/ml at a ratio of 200 vs 8.5±3.9 pg/ml with dermal microvascular EC alone, p=0.0091 paired t test). TSLP production by dermal microvascular EC was further increased in the presence of ADP-activated platelets compared to unstimulated platelets (318.2±105 pg/ml vs 164.7±41.4 pg/ml respectively, p=0.03 paired t test see Figure 5A). Noteworthy, TSLP production was dependent on the dermal microvascular EC since activated platelets were unable to secrete significant amount of TSLP. To decipher the mechanism responsible for platelet-induced TSLP production by dermal microvascular EC, we first blocked IL-1β using the monoclonal antibody canakinumab. Addition of canakinumab significantly reduced platelet-induced TSLP production (20.9±3.7 pg/ml vs 64.8 ±3.9 pg/ml, p<0.0001 paired t test, see Figure 5B), but not fully, suggesting that other platelet-derived factors could be also implicated. Because, platelet-derived serotonin has been directly involved in SSc pathogenesis (Dees et al. **([11])),** we added recombinant serotonin at different concentrations and observed that serotonin was able to induce TSLP production by dermal microvascular EC (58.9±11.4 pg/ml at 90 ng/ml vs 0.75±0.5 pg/ml for dermal microvascular EC alone respectively, p=0.013 paired t test, see Figure 5C). We did not observe any synergic effect of IL1β and serotonin in TSLP production. The addition of serotonin receptors (5HT2a and 5HT2b) blocking antibodies to the platelet/EC co-culture significantly reduced TSLP production (649±13.8 pg/ml in platelet/EC vs 335.1±28.5 pg/ml with canakinumab vs 242.2±9.1 pg/ml with canakinumab + serotonin receptor inhibitors, p=0.0072 ANOVA see Figure 5D). Altogether these results suggest that platelet-induced TSLP production by dermal microvascular EC is mainly due to IL-1β and serotonin to a lesser extent.

### TSLP induces fibrosis in human through extracellular matrix production and collagenase inhibition

TSLP has been implicated in the fibrotic process in mouse models, but its role in humans remains uncertain (Usategui et al. **([29]);** Christmann et al. **([30])).** We therefore assessed whether recombinant TSLP (rTSLP) could directly impact the activation and extracellular matrix (ECM) production of dermal fibroblasts from HD.

Specific TSLP receptor CRLF2 mRNA was detected in all normal fibroblasts (data not shown). Dermal HD fibroblasts were stimulated with IL-1β, TSLP or TGFβ as a control condition. As expected, TGFβ induced collagen1A1 (5.35±0.9 fold increase vs medium condition, p=0.0486, paired t-test see Figure 6A) and collagen1A2 (data not shown) mRNA overexpression in HD dermal fibroblasts. Interestingly, rTSLP induced a reproducible and significant increase of collagen1A1 (1.5±0.06 fold increase vs medium condition, p=0.0188, paired t-test see Figure 6A) and collagen1A2 (data not shown) mRNA expression by fibroblasts. Concomitantly, rTSLP significantly reduced the expression of the collagenase MMP-1 in the same extent to TGFβ1 (0.59±0.06 fold increase and 0.56±0.14 vs medium condition respectively, p=0.0241, paired t-test see Figure 6B). Conversely, the collagen/collagenase ratio was significantly increased in the presence of rTSLP demonstrating the direct pro-fibrotic effect of the cytokine (2.6±0.3 fold increase vs medium condition, p=0.0309, paired t-test see Figure 6C). Interestingly, in the presence of recTSLP, fibroblasts purified from TSLP+ fibrotic skin of two dcSSc patients have lower collagen/collagenase ratio compared to fibroblasts purified from TSLP- skin of a IcSSc patient or normal fibroblasts. We also tested the effect of IL-1β a cytokine potentially present in the SSc microenvironment. In contrast to TSLP, results with IL-1β were less homogeneous and did not reach statistical significance. IL-1β induced a slight increase of collagen expression (1.7±0.4 fold increase, p=0.193, see Figure 6A) but in sharp contrast to TSLP and TGFβ1, IL-1β increased MMP-1 expression (5.7±1.7 fold increase, p=1.1, see Figure 6B). Consequently, the collagen/collagenase ratio tend to diminish although non significantly (0.47±0.3 fold increase vs medium condition, p=0.2, paired t-test see Figure 6C). Noteworthy, rTSLP and TGF-β induced a strong down-regulation of MCP-1 mRNA expression (0.48±0.02 and 0.31±0.09 fold increase vs medium condition respectively, p=0.0012, see Figure 6D) whereas IL-1β highly increased the expression of MCP-1 in human dermal fibroblasts (52.7±11.2 fold increase vs medium condition respectively, p=0.0471, see Figure 6D)

Altogether, our findings demonstrate that TSLP promote a pro-fibrotic response in human dermal fibroblasts.

### DISCUSSION

A better characterization of fibrotic mechanisms involved in SSc is a keystone to develop biomarkers and effective disease-modifying therapies that are still an unmet medical need even though breakthrough have been recently made (van Bon L, Affandi AJ, Broen J, et al. Proteome-wide analysis and CXCL4 as a biomarker in systemic sclerosis. N Engl J Med 2014;370:433-443 (van Bon et al. **([10]) ;** Dees et al. **([11]) ;** Rice LM, Padilla CM, McLaughlin SR, et al. Fresolimumab treatment decreases biomarkers and improves clinical symptoms in systemic sclerosis patients. J Clin Invest 2015;125:2795-2807 **([32]) ;** Bhattacharyya S, Tamaki Z, Wang W, et al. FibronectinEDA promotes chronic cutaneous fibrosis through Toll-like receptor signaling. Sci Transl Med 2014; 6:232ra250 **([33]) ;** Wynn TA, Ramalingam TR. Mechanisms of fibrosis: therapeutic translation for fibrotic disease. Nat Med 2012;18:1028-1040 **([34])).** In this study, we provide evidences of a new pathogenic loop implicating activated platelets, microvascular endothelial cells and ECM production in SSc.

Our findings shed a new light on the numerous contributors to TSLP production in SSc and extend previous publications on the subject (Usategui et al. **([29]) ;** Christmann et al. **([30])).** Usategui *et al.* demonstrated that TSLP up-regulation was mainly attributed to keratinocytes, and in a lesser extend to dermal cells such as mast cells and fibroblasts, whereas the Lafyatis group (Christmann et al. **([30]))** did not observe any differences in keratinocyte staining between SSc patients and controls (Usategui et al. **([29]) ;** Christmann et al. **([30])).** Instead they observed a specific prominent perivascular staining attributed to perivascular infiltrating CD163+ macrophages in SSc patients. The precise nature of those discrepancies is not obvious, but might be linked to the population studied (dcSSc in the Lafyatis study vs dcSSc and IcSSc in ours), and particularly the stage of the disease as perivascular infiltrate including T cells and macrophages are classically observed in the early forms of the disease (Manetti M. Deciphering the alternatively activated (M2) phenotype of macrophages in scleroderma. Exp Dermatol 2015; 24:576-578 **([35])).**

Initially associated to allergy, deregulated expression of TSLP is now observed in a growing number of disorders including atopic dermatitis and psoriasis, idiopathic pulmonary fibrosis, rheumatoid arthritis as well as cancer and inflammation (Datta et al. **([25]) ;** Soumelis V, Reche PA, Kanzler H, et al. Human epithelial cells trigger dendritic cell mediated allergic inflammation by producing TSLP. Nat Immunol 2002;3:673-680 **([36]) ;** Volpe E, Servant N, Zollinger R, et al. A critical function for transforming growth factor-beta, interleukin 23 and proinflammatory cytokines in driving and modulating human T(H)-17 responses. Nat Immunol 2008;9:650-657 **([37]) ;** Zhou B, Comeau MR, De Smedt T, et al. Thymic stromal lymphopoietin as a key initiator of allergic airway inflammation in mice. Nat Immunol 2005;6:1047-1053 **([38])**). Noteworthy in those settings, TSLP-expressing EC are not classically observed. Furthermore the observation of TSLP association with digital ulcers and PAH, strongly suggests a direct link between the SSc-associated vasculopathy and TSLP production. As a consequence, endothelial cell-deregulated expression of TSLP appears more specific for SSc and might constitute a new biomarker particularly in early forms of the disease.

Platelets are not only the sentinels of vasculature integrity but also play a role in the modulation of innate as well adaptive immune cell responses (Boilard E, Blanco P, Nigrovic PA. Platelets: active players in the pathogenesis of arthritis and SLE. Nat Rev Rheumatol 2012;8:534-542 **([39]) ;** Elzey BD, Tian J, Jensen RJ, et al. Platelet-mediated modulation of adaptive immunity. A communication link between innate and adaptive immune compartments. Immunity 2003;19:9-19 **([40]) ;** Duffau P, Seneschal J, Nicco C, et al. Platelet CD154 potentiates interferon-alpha secretion by plasmacytoid dendritic cells in systemic lupus erythematosus. Sci Transl Med 2010;2:47ra63 **([41])**). Platelets activation has been described in SSc (Kahaleh et al. **([5]) ;** Postlethwaite et al. **([6]) ;** Solanilia et al. **([7]) ;** Chiang et al. **([8])**) but their contribution to fibrosis have only been demonstrated recently (Dees et al. **([11]) ;** Kowal-Bielecka et al. **([14])**). While platelet-derived beta-thromboglobulin and CxCL4 levels are increased in broncho-alveolar lavage from SSc patients with pulmonary interstitial fibrosis (Kowal-Bielecka et al. **([14])**), platelet-derived serotonin promotes directly fibroblasts activation and ECM production both in human and mouse model of SSc (Dees et al. **([11])**). Here, we show that platelet-derived IL-1β and to a lesser extent serotonin, induced TSLP production by microvascular endothelial cells in a dose-dependent manner. In turns, rTSLP reproducibly promotes a pro-fibrotic profile in normal fibroblasts, which is in line with the pro-fibrotic gene signature observed by Christmann *et al.* after continuous sub-cutaneous injection of TSLP in mice (Christmann et al. **([30])**).

In conclusion, we defined a new pathogenic loop involving, platelets, endothelial cells, and fibroblast in SSc patients, questioning the impact of blocking platelet activation in SSc to prevent fibrosis. In this line, the observation of a diminished fibrosis in two mice model of scleroderma treated by platelets anti-aggregant paves the road for new clinical trials in human SSc and particularly in early forms of the disease (Dees et al. **([11])).**

### Example 3: Clinical study in relation with the preventive treatment of SSc

A prospective phase II/III clinical trial is conducted at the University Hospital Center of Bordeaux. This trial is a multicentric, randomized, placebo controlled in parallel, double-blind assay. Ninety-nine subjects with Raynaud's phenomenon and a dysimmunity are randomized as follows:
- Arm 1 (66 subjects): Clopidogrel 75 mg, once a day, 24 months
- Arm 2 (33 subjects): Placebo, 75 mg, once a day, 24 months Patients belonging to groups I, II and III are included.
   - Group I is defined as the combination of a RP a specific dysimmunity (ie anti-ScI70, anti-centromere, anti-RNA pol III, anti-Th/To and/or anti-Pm-ScI) and abnormal capillaroscopy.
   - Group II, includes subjects with RP, specific dysimmunity without abnormal capillaroscopy.
   - Group III which associates RP, non-specific dysimmunity (anti-nuclear antibodies other than the aforementioned auto-Ab) and abnormal capillaroscopy

The main objective of the study is to assess in patients with high risk to develop a scleroderma the efficacy of a 2-year clopidogrel treatment taken as described above on preventing the occurrence of a systemic sclerosis clinical marker (according the ACR/EULAR 2013 classification criteria) within these 2 years.

A systemic sclerosis clinical marker, different from Raynaud's phenomenon and the dysimmunity present at the initiation of the assay can be: cutaneous sclerosis, digital ulcers, stage 3 or 4 dyspnea, heartburn resistant to a 1-month conventional treatment, telangiectasia, subcutaneous calcinosis, myositis.

A comparison between arm 1 and Arm 2 is done.

Moreover, the platelet activation level, the endothelial activation level and the circulating TSLP are assessed.

Patients will be followed once a quarter except during the first month of treatment.

Exams will be performed at month 1, 3, 6, 9, 12, 15, 18, 21 and 24, according to Table 2 below.

**Table 2:**

| | Selection | Inclusion | Month 1 | Month 3 | Month 6 | Month 9 | Month 12 | Month 15 | Month 18 | Month 21 | Month 24 | Supple Mentary visit |
|---|---|---|---|---|---|---|---|---|---|---|---|---|
| Inclusion criteria control | X | X | | | | | | | | | | |
| written informed consent | X | | | | | | | | | | | |
| randomization | | X | | | | | | | | | | |
| | | | | | | | | | | | | |
| linical examination: | | | | | | | | | | | | |
| - weight, size | | | | | | | | | | | | |
| - vital signals | | | | | | | | | | | | |
| - number of painfull joints | | | | | | | | | | | | |
| - number of joint swelling | | | | | | | | | | | | |
| - dyspnea | | | | | | | | | | | | |
| - pulmonary examination | | | | | | | | | | | | |
| - cardiac examination | | | | | | | | | | | | |
| - lower limb oedema | | | | | | | | | | | | |
| - Rodnan score | X | X | X | X | X | X | X | X | X | X | X | X |
| - EUSTAR score | | | | | | | | | | | | |
| - digital ulcers | | | | | | | | | | | | |
| - telangiectasias | | | | | | | | | | | | |
| - sub-cutaneous calcinosis | | | | | | | | | | | | |
| - evaluation of cutaneous | | | | | | | | | | | | |
| - muscular pain | | | | | | | | | | | | |
| - pyrosis, dysphagia | | | | | | | | | | | | |
| - diarrhea, constipation | | | | | | | | | | | | |
| - bleeding,haemorrhage | | | | | | | | | | | | |
| - medical history | | | | | | | | | | | | |
| - concomittant treatment | | | | | | | | | | | | |
| Patient questionnaire: | | | | | | | | | | | | |
| - Health Assessment Questionnaire | | | | | | | | | | | | |
| - Short Form (36) Health Survey | | X | | | | | X | | | | X | X |
| - Cochin's hand | | | | | | | | | | | | |
| - patient's global assessment of Disease Activity (PGA) | | | | | | | | | | | | |
| - Physician's Global Assessment of Disease Activity (MDGA) | | | | | | | | | | | | |
| Blood analysis: | | | | | | | | | | | | |
| - blood formula | | | | | | | | | | | | |
| - VS, CRP | | | | | | | | | | | | |
| - urea, creatinin | X | X | X | X | X | X | X | X | X | X | X | X |
| - ASAT/ALAT, gamma GT, bilirubin, alkaline phosphatases | | | | | | | | | | | | |
| Urea spot for protein/creatinin ratio | | X | | | | | X | | | | | |
| complement sera dosage (C3, C4, CH50) | | X | | | | | X | | | | | |
| ANA, anti ds-DNA auto-antibody | X | | | | | | | | | | | |
| Auto-antibody [Ro (SS-A), La (SS-B), RNP, Sm, SCL70, Centromere B, RNA polymerase III, JO1] | X | | | | | | | | | | | |
| Auto-antibody [Fibrillarine, Th/To, PDGFR, Ku, PM-SCL75, PM-SCL100, NOR-90, A centromere] | X | | | | | | | | | | | |
| NT, pro-BNP, CPK | | X | | | | | X | | | | | |
| Treatment administration | | X | X | X | X | X | X | X | X | X | X | X |
| Treatment instruction | | X | X | X | X | X | X | X | X | X | X | X |
| Biological samples collection: | | | | | | | | | | | | |
| - 1 EDTA tube | | | | | | | | | | | | |
| - 5 plasma samples | | X | | | X | | X | | X | | X | X |
| - 3 PBMC samples | | | | | | | | | | | | |
| - 5 sera samples | | | | | | | | | | | | |
| electrocardiogram | | X | | | | | X | | | | X | |
| thoracic tomodensimetry | | X | | | | | | | | | | |
| respiratory fonctional tests + DLCO | | X | | | | | X | | | | X | |
| cardiac echography | | X | | | | | X | | | | X | |
| capillaroscopy | | X | | | | | X | | | | X | |
| EI/EIG analysis | | X | X | X | X | X | X | X | X | X | X | X |

### Example 4: treatment of platelets from a healthy non sclerodermic patient treated with clopidogrel

Briefly, platelets purified from healthy normal subject or a non-sclerodermic patient treated with clopidogrel for preventing atherothrombotic events were co-incubated with human normal microvascular endothelial cells at a 200:1 ratio for 24 hrs. Cell culture supernatant was collected and TSLP content was measured using a dedicated commercial ELISA kit. As shown in figure 7, platelets from the patient treated by clopidogrel failed to induce TSLP production by endothelial cells. This result is not observed with platelets from healthy patients not treated by clopidogrel.

The endothelial TSLP production is not inhibited by healthy patients platelets treated with acetylsalicylic acid (aspirin active metabolite) (see Figure 8).

These results show that blockade of the pro-fibrotic TSLP molecule expression *in vitro* from healthy endothelial cells is not observed with all the anti-aggregating agents. This leads to the conclusion that the inhibitory effect of clopidogrel could be due to its immunomodulatory effect, in addition to its specific effect on P2Y12 receptor and its anti-aggregating effect. Indeed, this effect is not observed with aspirin.

### REFERENCES

**1.** Gabrielli A, Avvedimento EV, Krieg T. Scleroderma. N Engl J Med 2009;360:1989-2003.
**2.** Tyndall et al.: "Causes and risk factors for death in systemic sclerosis· a study from the EULAR Scleroderma Trials and Research (EUSTAR) database", Ann Rheum Dis 2010; 69: 1809-1815.
**3.** Pattanaik D, Brown M, Postlethwaite BC, et al. Pathogenesis of Systemic Sclerosis. Front Immunol 2015;6:272.
**4.** Prescott RJ, Freemont AJ, Jones CJ, et al. Sequential dermal microvascular and perivascular changes in the development of scleroderma. J Pathol 1992;166:255-263.
**5.** Kahaleh MB, Osborn I, Leroy EC. Elevated levels of circulating platelet aggregates and beta-thromboglobulin in scleroderma. Ann Intern Med 1982;96:610-613.
**6.** Postlethwaite AE, Chiang TM. Platelet contributions to the pathogenesis of systemic sclerosis. Curr Opin Rheumatol 2007;19:574-579.
**7.** Solanilla A, Villeneuve J, Auguste P, et al. The transport of high amounts of vascular endothelial growth factor by blood platelets underlines their potential contribution in systemic sclerosis angiogenesis. Rheumatology (Oxford) 2009;48:1036-1044.
**8.** Chiang TM, Takayama H, Postlethwaite AE. Increase in platelet non-integrin type I collagen receptor in patients with systemic sclerosis. Thromb Res 2006;117:299-306.
**9.** Maugeri N, Franchini S, Campana L, et al. Circulating platelets as a source of the damage-associated molecular pattern HMGB1 in patients with systemic sclerosis. Autoimmunity 2012;45:584-587.
**10.** van Bon L, Affandi AJ, Broen J, et al. Proteome-wide analysis and CXCL4 as a biomarker in systemic sclerosis. N Engl J Med 2014;370:433-443.
**11.** Dees C, Akhmetshina A, Zerr P, et al. Platelet-derived serotonin links vascular disease and tissue fibrosis. J Exp Med 2011;208:961-972.
**12.** Allanore et al.: "Increased plasma soluble CD40 ligand concentrations in systemic sclerosis and association with pulmonary arterial hypertension and digital ulcers", Ann Rheum Dis 2005; 64: 481-483.
**13.** Yoshizaki et al.: "Clinical significance of serum HMGB-1 and sRAGE levels in systemic sclerosis: association with disease severity", J Clin Immunol 2009;29: 1 80-189.
**14.** Kowal-Bielecka et al.: "Beta thromboglobulin and platelet factor 4 in bronchoalveolar lavage fluid of patients with systemic sclerosis", Ann Rheum Dis 2005;64:484-486.
**15.** Klimiuk et al.: "Platelet serotonin in systemic sclerosis", Ann Rheum Dis 1989;48:586-589.
**16.** Pauling et al.: "The contribution of platelets to the pathogenesis of Raynaud's phenomenon and systemic sclerosis", Platelets 2013; 24: 503-515.
**17.** Silveri et al.: "Relative rates of endothelial cell damage and platelet activation in primarv Raynaud's phenomenon (RP) and RP secondary to systemic sclerosis", Scand J Rheumatol 2001 ; 30: 290-296.
**18.** Vettori et al.: "Early systemic sclerosis: serum profiling of factors involved in endothelial, T-cell, and fibroblast interplay is marked by elevated interleukin-33 levels", J Clin Immunol 2014; 34: 663-668.
**19.** Hillen MR, Radstake TR, Hack CE, et al. Thymic stromal lymphopoietin as a novel mediator amplifying immunopathology in rheumatic disease. Rheumatology (Oxford) 2015.
**20.** Lo Kuan E, Ziegler SF. Thymic stromal lymphopoietin and cancer. J Immunol 2014;193:4283-4288.
**21.** Ito T, Wang YH, Duramad O, et al. TSLP-activated dendritic cells induce an inflammatory T helper type 2 cell response through OX40 ligand. J Exp Med 2005;202:1213-1223.
**22.** Pedroza-Gonzalez A, Xu K, Wu TC, et al. Thymic stromal lymphopoietin fosters human breast tumor growth by promoting type 2 inflammation. J Exp Med 2011;208:479-490.
**23.** van den Hoogen F, Khanna D, Fransen J, et al. 2013 classification criteria for systemic sclerosis: an American College of Rheumatology/European League against Rheumatism collaborative initiative. Arthritis Rheum 2013;65:2737-2747.
**24.** Truchetet ME, Brembilla NC, Montanari E, et al. Interleukin-17A+ cell counts are increased in systemic sclerosis skin and their number is inversely correlated with the extent of skin involvement. Arthritis Rheum 2013;65:1347-1356.
**25.** Datta A, Alexander R, Sulikowski MG, et al. Evidence for a functional thymic stromal lymphopoietin signaling axis in fibrotic lung disease. J Immunol 2013; 191:4867-4879.
**26.** Brembilla NC, Montanari E, Truchetet ME, et al. Th17 cells favor inflammatory responses while inhibiting type I collagen deposition by dermal fibroblasts: differential effects in healthy and systemic sclerosis fibroblasts. Arthritis Res Ther 2013;15: R151.
**27.** Yoda A, Yoda Y, Chiaretti S, et al. Functional screening identifies CRLF2 in precursor B-cell acute lymphoblastic leukemia. Proc Natl Acad Sci U S A 2010;107:252-257.
**28.** Le QT, Gomez G, Zhao W, et al. Processing of human protryptase in mast cells involves cathepsins L, B, and C. J Immunol 2011;187:1912-1918.
**29.** Usategui A, Criado G, Izquierdo E, et al. A profibrotic role for thymic stromal lymphopoietin in systemic sclerosis. Ann Rheum Dis 2013.
**30.** Christmann RB, Mathes A, Affandi AJ, et al. TSLP upregulation in human SSc skin and induction of overlapping profibrotic genes and intracelullar signaling with IL-13 and TGFss. Arthritis Rheum 2013.
**31.** Loppnow H, Bil R, Hirt S, et al. Platelet-derived interleukin-1 induces cytokine production, but not proliferation of human vascular smooth muscle cells. Blood 1998;91:134-141.
**32.** Rice LM, Padilla CM, McLaughlin SR, et al. Fresolimumab treatment decreases biomarkers and improves clinical symptoms in systemic sclerosis patients. J Clin Invest 2015;125:2795-2807.
**33.** Bhattacharyya S, Tamaki Z, Wang W, et al. FibronectinEDA promotes chronic cutaneous fibrosis through Toll-like receptor signaling. Sci Transl Med 2014;6:232ra250.
**34.** Wynn TA, Ramalingam TR. Mechanisms of fibrosis: therapeutic translation for fibrotic disease. Nat Med 2012;18:1028-1040.
**35.** Manetti M. Deciphering the alternatively activated (M2) phenotype of macrophages in scleroderma. Exp Dermatol 2015;24:576-578.
**36.** Soumelis V, Reche PA, Kanzler H, et al. Human epithelial cells trigger dendritic cell mediated allergic inflammation by producing TSLP. Nat Immunol 2002;3:673-680.
**37.** Volpe E, Servant N, Zollinger R, et al. A critical function for transforming growth factor-beta, interleukin 23 and proinflammatory cytokines in driving and modulating human T(H)-17 responses. Nat Immunol 2008;9:650-657.
**38.** Zhou B, Comeau MR, De Smedt T, et al. Thymic stromal lymphopoietin as a key initiator of allergic airway inflammation in mice. Nat Immunol 2005;6:1047-1053.
**39.** Boilard E, Blanco P, Nigrovic PA. Platelets: active players in the pathogenesis of arthritis and SLE. Nat Rev Rheumatol 2012;8:534-542.
**40.** Elzey BD, Tian J, Jensen RJ, et al. Platelet-mediated modulation of adaptive immunity. A communication link between innate and adaptive immune compartments. Immunity 2003;19:9-19.
**41.** Duffau P, Seneschal J, Nicco C, et al. Platelet CD154 potentiates interferon-alpha secretion by plasmacytoid dendritic cells in systemic lupus erythematosus. Sci Transl Med 2010; 2: 47ra63.
**42.** Koenig et al.: "Autoantibodies and Microvascular Damage Are Independent Predictive Factors for the Progression of Raynaud's Phenomenon to Systemic Sclerosis », ARTHRITIS & RHEUMATISM, Vol. 58, No. 12, December 2008, pp 3902-3912.
**43.** Valentini et al.: "Early Systemic Sclerosis: Analysis of the Disease Course in Patients With Marker Autoantibody and/or Capillaroscopic Positivity", Arthritis Care & Research, Vol. 66, No. 10, October 2014, pp 1520-1527.
**44.** Jean-Pierre Maffrand: "The story of clopidogrel and its predecessor, ticlopidine: Could these major antiplatelet and antithrombotic drugs be discovered and developed today?", C. R. Chimie 15 (2012) 737-743.
**45.** Agmon-Levin N. et L.: "International recommendations for the assessment of autoantibodies to cellular antigens referred to as anti-nuclear antibodies.", Ann Rheum Dis. 2014 Jan;73(1):17-23.

## Claims

1. Substance that inhibits P2Y12 receptor for use in the preventive treatment of systemic sclerosis in patients with Raynaud's phenomenon and a dysimmunity.

2. Substance for use according to claim 1, wherein said substance is selected among a thienopyridine, ticagrelor, elinogrel and cangrelor, their active metabolites, a pharmaceutical salt thereof and a mixture thereof.

3. Substance for use according to claim 1 or 2, wherein said substance is selected among a thienopyridine, its active metabolites and pharmaceutical salts thereof.

4. Substance for use according to anyone of claim 1 to 3, wherein said thienopyridine is selected among clopidogrel, prasugrel and ticlopidine.

5. Substance for use according to anyone of claims 1 to 4, wherein said dysimmunity is a presence of antinuclear antibodies.

6. Substance for use according to claim 5, wherein said antinuclear antibodies are one or more antibodies selected among anti-ScI70, anti-centromere, anti-RNA polymerase III, anti-Th/To and anti-PM-ScI antibodies.

7. Substance for use according to anyone of the preceding claims, wherein said patients also present a capillaroscopy with scleroderma pattern.

8. Substance for use according to anyone of the preceding claims, wherein said pharmaceutical salt is chosen in the group comprising hydrogen sulphate, besylate, bisulfate, hydrochloride, resinate, napadisilate, hydrochloride, hydrobromide, phosphate, potassium and nitrate.

9. Substance for use according to anyone of the preceding claims, wherein said substance is hydrogen sulphate clopidogrel.

10. Substance for use according to anyone of the preceding claims, by administration of a dose of said substance comprised of from 5 mg to 250 mg per day.

11. Substance for use according to anyone of the preceding claims, wherein said preventive treatment consists in delaying or preventing the onset of systemic sclerosis in the patients.

12. Substance for use according to anyone of the preceding claims, wherein said preventive treatment results in a lack, within 2 years after the start of the treatment, of at least one symptom chosen in the group comprising cutaneous sclerosis, digital ulcer, dyspnea at stage 3 or 4, abnormal capillaroscopy, heartburn resisting to one month of a conventional treatment, joint pain and/or joint swelling, telangiectasias, sub-cutaneous calcinosis, pigmentation disorders and myalgia.

13. Substance for use according to anyone of the preceding claims, for inhibiting platelet activation.

14. Substance for use according to anyone of the preceding claims, for inhibiting the expression and secretion of pro-inflammatory factors resulting from platelet activation.

15. Substance for use according to anyone of the preceding claims, for inhibiting the expression and/or production of Thymic Stromal Lymphopoietin (TSLP) by endothelial cells.

16. Substance for use according to anyone of the preceding claims, by oral administration of said substance.
